(19) Europäisches Patentamt
European Patent Office
Office européen des brevets

(11) Publication number: **0 157 960**
**A1**

(12) # EUROPEAN PATENT APPLICATION

(21) Application number: 84302059.5

(22) Date of filing: 27.03.84

(51) Int. Cl.[4]: A 61 L 15/00

(43) Date of publication of application:
16.10.85 Bulletin 85/42

(84) Designated Contracting States:
AT BE CH DE FR GB IT LI LU NL SE

(71) Applicant: PERSONAL PRODUCTS COMPANY
Van Liew Avenue
Milltown New Jersey 08850(US)

(72) Inventor: Korpman, Ralf
885 Bluestone Lane
Bridgewater New Jersey(US)

(72) Inventor: Gandy, Charles
415 Matchaponix Road
Jamesburg New Jersey(US)

(74) Representative: Jones, Alan John et al,
CARPMAELS & RANSFORD 43 Bloomsbury Square
London, WC1A 2RA(GB)

(54) **Dispersed absorbent products and method of use.**

(57) An absorbent product comprising a water insoluble, water swellable absorbent for aqueous fluids in particulate form dispersed in an inert organic vehicle is described. Typical absorbents include acrylate polymers, acrylate polymer modified polysaccharides, cross-linked carboxymethylcellulose, cross-linked poly(alkylene oxides) and certain gum blends. The vehicles include oils, liquid resins, liquid rubbers and liquid polyalkenes, glycol ethers and higher alcohols. The product may be employed alone or on a substrate.

EP 0 157 960 A1

Croydon Printing Company Ltd

0157960

Dispersed Absorbent Products and Method of Use

The present invention relates to absorbent products, more particularly to absorbent products in a novel form adaptable for more facile and controlled application in the production of absorbent articles and in the preparation of superior absorbent articles.

Absorbent materials having superior capacity for immobilizing water and aqueous fluids, and frequently referred to in the art as "superabsorbents", "hydrogels" or "hydrocolloids", and available in particulate, i.e., pulverulent or granular form, have been incorporated in the cellulosic absorbent structure of diapers, sanitary napkins and other articles to increase their absorptive efficiency. Uniform incorporation and retention of these particulate absorbent materials have not been readily accomplished. Even when uniformly distributed, the dry particulate materials have been found to shift, and special constructions have been employed to retain the particles in place. It is desirable to provide a product which permits facile incorporation of absorbent particulate materials and more uniform retention in absorbent articles.

The present invention is directed to a novel absorbent product useful alone or as a simple, convenient means for supplying particulate absorbent to articles and for

retaining them thereon, whether the absorbent be of materials known in the art as superabsorbents or of other particulate materials having absorbent properties. As used herein, the liquid contemplated to be absorbed is water or aqueous liquid while the carrier of the particulate water soluble, water swellable absorbent and component of the composition is an organic vehicle. The product is especially useful for conveniently placing absorbent materials in bandages and in articles such as diapers, sanitary napkins and absorbent sheets for many sanitary, health care, consumer and industrial applications.

By "absorbent product" is meant a composition comprising a particulate water insoluble, water swellable absorbent having a gel capacity of at least 10 as hereinafter defined dispersed in an inert organic vehicle as hereinafter defined. The composition is heterogeneous in that the particulate matter is suspended and not dissolved in the vehicle. The product can be coated or otherwise applied to a substrate to provide a superior article in which the particulate absorbent is uniformly dispersed and is retained in place, and in which there is no significant diminution in absorption capabilities as might be expected as a result of the coating of the surfaces of the particulate absorbents by the organic vehicle which is generally water-immiscible. Moreover, whether employed alone or on a substrate, absorption of water is substantially instantaneous. In addition, the product after absorption of water is resistant to loss of water when subjected to pressure.

When polyethylene glycol is used as the vehicle, the absorbent is blended therewith. The purpose of this blending is to provide particles capable of being retained in fibrous mats or other absorbent matrices. The blending also reduces dusting of the powder and shake-out losses from the fiber matrices.

By the expression "absorbent" is meant a material having an enhanced capacity for removing water or aqueous fluids such as physiological saline, serum, blood, mucous, urine, and other body fluids, as well as salt, fertilizer and other solutions not necessarily associated with living processes. The expression is more specifically directed to materials having absorptive properties superior to natural cellulose or starch, and which may be chemically modified cellulose or starch or may be wholly synthetic. The most common and best known of such materials currently available are acrylate polymer modified polysaccharides and synthetic acrylate polymers as hereinafter defined; other materials include cross-linked carboxymethylcellulose, cross-linked poly(alkylene oxide), gums, and gum blends. The absorptive capacity of the materials contemplated is at least 10 times and up to 1500 times or more the weight of the material in dry form, and commonly about 15 times to about 70 times the weight or more.

By "particulate" is meant a substance in the form of fine discrete particles. They may be variously shaped such as spherical, rounded, angular, acicular, irregular, fragmented and the like. The particles may range in size from about 1 micron to 2 x $10^4$ microns in diameter or cross-section (largest dimension when not spherical). Particulate is also intended to include absorbents in the form of fibers of short lengths. The particles may be otherwise designated as granular or powder. By "powder" is meant particles of particle size of from about 1 to $10^3$ microns.

By the expression "inert" is meant a vehicle which is non-reactive with the water or moisture, with the absorbent or with the substrate on which the product may be applied if employed as coating.

By "low-viscosity" is meant having a viscosity at temperatures of use of no greater than about 100,000 centipoises, preferably in the range of from about 25 to 5,000 centipoises. Preferably, when the vehicle contemplated is a liquid, it exhibits the foregoing viscosity at ambient temperatures and for most part, use at ambient temperatures is contemplated.

The absorbent materials are polymers in which hydrophilic groups constitute at least 25 percent and up to 72 percent of the molecular structure and in which the polymeric network is usually but not necessarily lightly cross-linked. Suitable synthetic absorbent polymers are modified natural polymers, e.g., modified polysaccharides, are generally water-insoluble but water-swellable. Certain natural gums although frequently classified as water-soluble also may be suitable provided they exhibit properties hereinafter detailed. Many of the suitable materials are those which have been reported to have an average molecular weight per cross-linkage in the range of from about 25,000 to about 300,000. The preferred polymers have an acrylate group in their molecular structure and are referred to herein as "acrylate absorbent". They may be completely synthetic acrylate polymers or acrylate modified polysaccharides, e.g., acrylate modified starch or acrylate modified cellulose. "Acrylate polymers", "polyacrylate" or "acrylate" embraces not only polymers which contain acrylate salt groups but those which also contain an acrylamide, acrylic acid or acrylic ester group. The absorbents may have another type of hydrophilic group in the structure such as sulfonate, oxide, etc.; such absorbents include sulfonated polystyrene, poly-(alkylene oxide), and the like. Carboxy-methylcellulose after cross-linking is also within the scope of absorbents as are gums and gum blends. The absorbents may be prepared by methods described in the literature.

Representative methods are hereinafter described. Also, most absorbents are available commercially.

The suitable absorbent polymers may be prepared by cross-linking a preformed water-soluble straight chain polymer, by polymerizing an appropriate monomer or a monomer and a co-monomer to simultaneously effect polymerization and cross-linking, or by incorporating a hydrophilic group into a completed polymer. An example of the latter is sulfonation of polymers to obtain polymers bearing sulfonic moieties. Moreover, where it is desired to have the salt form of the carboxylic or sulfonic acid group, the polymer may be prepared first as an acid, ester, amide, or nitrile and the product hydrolyzed in whole or in part.

The preferred synthetic acrylate absorbents are those which have a salt group, an acid group or which have both an amide group and a salt or acid group and which have been represented in the literature, e.g., U.S. Patent 3,686,024, by the following formula:

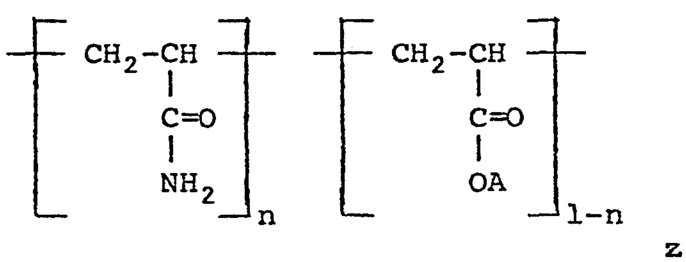

where A is an alkali metal ion such as sodium or potassium, or is hydrogen, n is from about 0.5 to about 0.9, 1-n defines the extent of hydrolysis, and z is the number of mer units between cross-links.

The acrylate absorbent containing both amide and carboxylate groups may be prepared either (1) by aqueously polymerizing about 2 to 20 weight percent acrylamide with from about 0.005 to 0.5 mole percent "based on acrylamide" of a

difunctional organic cross-linker such as N,N'-methylenebisacrylamide in the presence of a free radical catalyst to obtain a water-swellable cross-linked polyacrylamide, and thereafter partially hydrolyzing in aqueous alkali to obtain a cross-linked polymer having both an amide and an alkali metal carboxylate groups as more fully described in U.S. Patent 3,247,171, or (2) by cross-linking a previously prepared linear polyacrylamide with a cross-linking compound such as N,N'-methylenebisacrylamide, 1,4-divinylbenzene, diallylamine, N,N-diallylmethacrylamide and the like, and thereafter hydrolyzing. A polymer having both an amide and a carboxylate group also may be prepared by copolymerizing acrylamide and acrylic acid alkali metal salt in the presence of a cross-linking monomer such as N,N'- methylenebisacrylamide in the presence of a catalyst system such as equal parts of ammonium persulfate and β-dimethylaminopropionitrile, also described in the aforesaid patent.

Acrylate absorbents with carboxylate groups only as functional groups may be prepared by cross-linking either by subjecting a monovalent cation salt of acrylic acid in water to the influence of high energy ionizing radiation as described in U.S. Patent 3,229,769 or by subjecting it to chemical cross-linking as described in British Patent 719,330.

Another type of absorbent is polysaccharide modified by having hydrophilic chains grafted thereon. By "hydrophilic chains" is meant a polymer chain obtained from monomers which have a group which is water-soluble or becomes water-soluble on hydrolysis, e.g., carboxyl, sulfonic, hydroxyl, amide, amino, quaternary ammonium and hydrolysis products thereof. Representative modified polysaccharides are described in U.S. Patent 4,076,663. Preferred polysaccharides have chains terminated by a carboxylate

group or mixture of carboxylate and amide groups. The modified polysaccharides are sometimes referred to in the literature as starch or cellulose graft copolymers; the preferred modified graft copolymers are polysaccharide acrylate polymers, i.e. starch acrylate polymer or cellulose acrylate polymer.

In the polysaccharide acrylate polymers, the hydrophilic chain is attached to the backbone of the cellulose or starch molecule through a carbon linkage. Thus, an acrylate modified cellulose may be represented by the formula

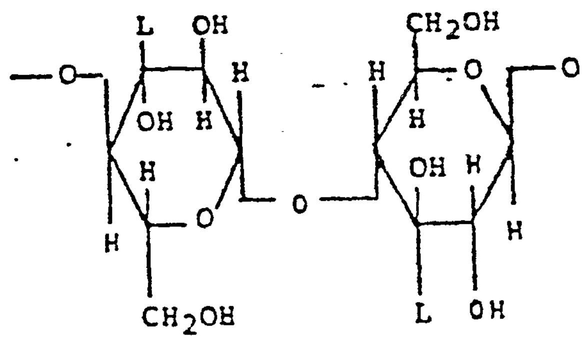

wherein L represents a hydrophilic chain of the general formula

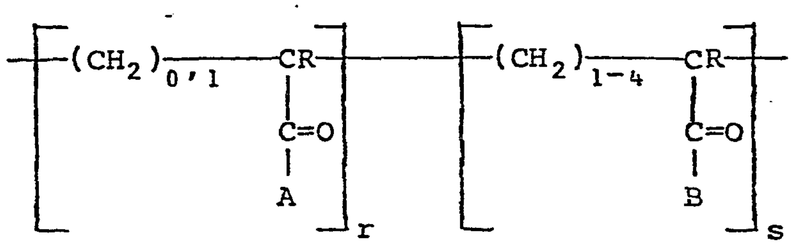

wherein $-\overset{\overset{O}{\|}}{C}-A$ and $-\overset{\overset{O}{\|}}{C}-B$ independently represents an acid, ester, alkali metal salt, ammonium salt, or amide group, each R independently is hydrogen or lower alkyl, r is an integer of from 0 to about 5000 and s is an integer of from 0 to about 5000, and r plus s is at least 500. An acrylate modified starch would be similar but would have a starch backbone.

The polysaccharide acrylates may be prepared, for example, by polymerizing an appropriate polysaccharide with acrylonitrile or methacrylonitrile, with methyl or ethyl acrylate, with acrylic or methacrylic acid, or with acrylamide or methacrylamide, and thereafter hydrolyzing the resulting polymer in whole or in part with aqueous alkali. Alternatively, they may be prepared by polymerizing an alkali metal salt of acrylic or methacrylic acid.

The procedures for carrying out graft-copolymerization of olefinically unsaturated chains onto cellulose and starch are well known in the art. Grafting of the hydrophilic material onto a starch or cellulose backbone can be accomplished simultaneously with the formation of the hydrophilic polymeric material in an aqueous medium, because the peroxide catalyst used to copolymerize the hydrophilic group producing monomers forms a redox catalyst system in combination with a reducing agent to effect chain transfer onto the starch or cellulose backbone. Reducing agents which may be employed include cerous, ferrous, cobaltous, and cuprous salts and the like. Graft copolymerization of olefinically-unsaturated chains also may be effected by irradiation (ultraviolet-, gamma-, or X-radiation) or by heating in an aqueous medium in the presence of an emulsifier.

In carrying out the operation, powdered starch or fibrous or pulpy cellulose is slurried in water containing a graft copolymerization catalyst system and the hydrophilic monomeric acrylate is added to the slurry and polymerized in situ at ambient temperature or above depending on the catalyst employed to obtain an acrylate modified polysaccharide. In this operation, a portion of the simultaneously formed hydrophilic polymer also may be physically entrapped into the polysaccharide backbone material during the polymerization process. The

graft-copolymers produced are recovered and dried at atmospheric pressure to obtain a relatively stiff and brittle material which may be comminuted to the desired particle size.

Other methods for producing graft copolymers may be found in U.S. Patent 2,922,768, 3,526,372, 3,661,815, 3,935,099, 4,028,290, 4,076,663 and 4,105,033.

Suitable polysaccharide acrylates are those in which the hydrophilic chain loading on the backbone is within the range of from about 10 percent by weight to about 90 percent by weight, preferably from about 40 to about 80 percent by weight of the graft copolymer.

In addition to modified natural and regenerated polymers, hydrocolloid particle components may comprise wholly synthetic hydrophilic particles. Examples of those now known in the art are polyacrylonitrile fibers which may be modified by grafting moieties thereon such as polyvinyl alcohol chains, polyvinyl alcohol itself, hydrophilic polyurethane, poly(alkyl phosphonates), partially hydrolyzed polyacrylamides (e.g., poly(N-N-dimethy acrylamide), sulfonated polystyrene, or a class of poly(alkylene oxide). These highly hydrophilic synthetic polymers may be modified by other chemical treatments such as cross-linking or hydrolysis. Further examples known in the art are the non-ionic hydrophilic polymers such as polyoxyethylene, polyoxypropylene and mixtures thereof which have been suitably cross-linked, either chemically or by irradiation. Still another more recent type is a derivative of isobutylene-maleic anhydride copolymer.

Another type of absorbent which may be a component of the liquid dispersed absorbent product of the present invention is cross-linked carboxymethylcellulose (CMC). Suitable cross-linked carboxymethylcellulose are those which

may be prepared by heat treatment or wet cross-linking of a water-soluble alkali metal salt of carboxymethylcellulose having from about 0.5 to about 1 carboxymethyl group for each anhydroglucose unit. In wet cross-linking, reagents bifunctional with respect to cellulose such as epichlorohydrin may be employed. A representative method for the preparation of cross-linked carboxymethylcellulose absorbent is heating a suitable alkali metal salt of carboxymethylcellulose in particulate form to a temperature between about 130°C and about 210°C for from a few minutes to several hours to obtain products having a new capacity of absorbing water as more fully described in U.S. Patent 2,639,239.

Still another type of absorbent which may be advantageously employed in the product in the present invention is cross-linked poly(alkylene oxide) of molecular weight of at least 100,000 which may be prepared by contacting poly(alkylene oxide) with a suitable cross-linking agent in the presence of free radical catalyst in a liquid medium at a temperature sufficient to effect the cross-linking, wherein the liquid medium is a solvent-nonsolvent mixture for poly(alkylene oxide) in which the nonsolvent portion is at least 35 percent by weight. The process is described in U.S. Patent 3,956,224.

Alternatively, poly(alkylene oxide) may be cross-linked by ionizing radiation as described in U.S. Patent 3,264,202, or co-cross-linked with at least one other water-soluble polymer by exposing aqueous systems of polymers to high energy radiation to produce cross-linked water-insoluble absorbent polymers as more fully described in U.S. Patent 3,957,605 and 3,898,143.

Still another type of absorbent are organic substances of polysaccharide character, e.g., natural or synthetic gums,

which must be employed as a blend. Typical gums which may be employed include locust bean gum, guar gum, xantham gum, tragacanth gum, karaya gum and the like. When blended, the polysaccharides appear to interact to provide absorbents having the swellability without the undesired solubility.

The absorbents prepared by the foregoing methods are generally obtained as granular solids. These solids may then be comminuted to the appropriate size. Preferably they are employed in the form of powder as previously defined.

In the instance wherein the vehicles suitable in the absorbent product of the present invention are liquid, they are not limited by chemical classification provided they are characterized by the viscosity and inertness previously detailed.

One type of suitable liquid vehicle is oil. The oils are primarily hydrocarbon, aromatic or paraffinic, preferably mineral oil, and for many applications that of the quality available as baby oil. Plant oils, i.e., glycerides of the appropriate viscosity range, may be employed but may be less desirable for other reasons.

Another type of liquid vehicle is liquid resin. By "liquid resin" is meant polymers in which the component monomers have been obtained from plants, petroleum or coal tar, and include polymerization products of "essential" oil of plants and rosin esters obtained by distillation of volatile components of plants. The polymers are those having a softening point of about 25°C or below. Suitable liquid resins include polymers which are primarily polymerization products of coumarone and indene, piperylene and isoprene, mixed olefins of predominantly $C_5$ olefin or

$C_9$ olefins, and rosin esters, especially of glycerol, ethylene glycol and pentaerythritol. They are available commercially under various trade names.

Another type of suitable liquid vehicle is liquid rubber. By "liquid rubber" is meant materials identifiable chemically with solid rubbers but which are liquid at ambient temperatures. These materials can be cured to form products having elastic properties; however, in the context of their use in the present invention, the materials do not exhibit elastic properties. Suitable liquid rubbers include low molecular weight polyisoprene, poly(styrene-butadiene), polybutadiene, and the like.

Still another class of suitable liquid vehicles are certain liquid polyalkenes. Particularly useful are polybutenes, e.g., polybutylenes and polyisobutylenes.

Yet another class of suitable vehicles are glycol ethers and high-boiling non-reactive hydroxylated compounds. Suitable glycol ethers are polyethylene glycol and polypropylene glycol in the molecular weight range of about 200 to about 600.

By "high-boiling non-reactive hydroxylated compounds" is meant to embrace alcohols and glycols which boil higher than 100°C and in which the absorbent may be dispersed without gel formation resulting. They include $C_5$ and higher alcohols, butylene and higher alkylene glycols and the like.

In the absorbent products, the relative amounts of absorbent and vehicle are dependent on the level of absorption required in the ultimate environment and on the viscosity of the liquid vehicle. Thus, if the absorption is to be accomplished by an article which has been

obtained by coating the product of the present invention to a component of the article as substrate, such as a pad as the component of a diaper as the article, the amount of the absorbent in the vehicle will be an amount sufficient to supply the desired absorptive capacity to the pad substrate and a vehicle will be selected which will impart suitable spreading characteristics for supplying such amounts of absorbent. However, if the absorbent product is to be applied directly to the area where aqueous fluid is to be removed without substrate, the absorbent product will generally have a higher content of absorbent. Generally from about 2 parts to 1000 parts, preferably about 25 parts to 100 parts, by weight of absorbent in 100 parts by weight of liquid vehicle may be employed. When the vehicle is polyethylene glycol, 200 parts to 1000 parts and preferably about 400 parts by weight of absorbant in 100 parts by weight of polyethylene glycol, may be employed.

The use of polyethylene glycol eliminates the sponge-cake effect or caking of blends produced by more hydrophilic materials.

The product may be modified to include other materials. Small quantities may be added to affect its spreading properties. Thus, if small quantities of absorbent is employed but a high viscosity liquid product is desired, the viscosity characteristic may be changed by adding viscosity modifiers, usually silicated powders at very low concentrations.

The absorbent product may be employed in the preparation of absorbent articles such as diapers, incontinent pads, sanitary napkins, bandages, and the like. In the preparation of absorbent articles, the absorbent product is applied to a substrate. The substrate most preferably is

a material having some liquid drawing property such as cellulosic materials and synthetic absorbent materials and includes materials which have this property conferred on it mechanically such as by being woven. However, a preferred embodiment is a nonwoven fabric such as a high loft, low density polyester nonwoven. When a liquid dispersed absorbent product is coated on a substrate, the latter, if it has liquid drawing properties, partially draws the liquid vehicle leaving the absorbent uniformly dispersed on the surface. While most of the liquid vehicle becomes drawn or penetrates into the substrate, a sufficient amount appears to remain in contact with the absorbent and the absorbent is not readily susceptible to mechanical displacement. If the substrate does not have drawing properties, the product is most preferably employed in a laminate in which a material having drawing properties is superposed on the liquid-dispersed absorbent product after it has been coated on the substrate.

When the vehicle is polyethylene glycol, the absorbent product is preferably applied to the desired substrate (which comprises an absorbent matrix) by air conveying or air blasting the product into the interstices of the absorbent matrix.

One application for the liquid-dispersed absorbent product is in the preparation of an improved diaper or similar absorbent article. In such application, preferably a thin layer of cellulosic batting has applied thereto the absorbent product of the present invention to produce a modified batting and the modified batting used to prepare a disposable diaper in a conventional way i.e., it is provided with a moisture impermeable backing sheet and a moisture permeable top sheet. Alternatively, a highly absorptive diaper or sanitary napkin may be prepared by providing select portions of a thicker batting with

concentrated areas of absorbent product. The article prepared by employing the absorbent product of the present invention differs from certain previously reported articles containing pulverulent absorbent in that the absorbent is substantially free of the shifting usually encountered when dry absorbent powder is incorporated. Moreover, the product simplifies incorporation of absorbent and renders unnecessary the specialized techniques and structures for placing and retaining absorbents in the batting to overcome mobility of absorbent in the batting.

In a modification of a diaper, bed pad or napkin the absorbent product may be applied to a conventional nonabsorbent moisture impermeable diaper backing and thereafter laminated with a conventional moisture permeable top sheet.

Another application for the absorbent product is in the preparation of bandages. For such application, the absorbent product may be applied directly to the gauze surface to produce a bandage of improved absorptive quality.

The liquid-dispersed absorbent products of the present invention, of viscosity less than about 5,000 centipoises, are especially useful as water scavangers and for immobilizing stray water. When the product is of very low viscosity, the product spreads substantially instantaneously with concomitant absorption and fixation of the water.

By incorporating additives such as wetting agents, surfactants, fertilizers, nematocides, insecticides, soil conditioning agents and the like, to the liquid-dispersed absorbent product as above described, compositions

suitable for use as soil amendments may be obtained which are more readily dispersed than dry mixtures.

Furthermore, additives such as medicinal agents, antibacterial agents and anti-odor agents may be incorporated into the absorbent products of the invention which are used for application to diapers and similar absorbent articles and to bandages.

The following examples illustrate the invention but are not to be construed as limiting.

EXAMPLES I-IX

Liquid-dispersed absorbent products having the compositions set forth in Table A are prepared.

TABLE A

| Component | Amount of Components (parts by weight) Examples I | II | III | IV | V | VI | VII | VIII | IX |
|---|---|---|---|---|---|---|---|---|---|
| **Absorbent** | | | | | | | | | |
| Starch polyacrylate +, (SGP 502S, Henkel) | 50 | | | | | | | | |
| Starch Polyacrylate +, (Stasorb*, A. E. Staley) | | 10 | | | | | | | |
| Cellulose Polyacrylate | | | 75 | | | | | | |
| Cross-linked Carboxymethylcellulose, (Aqualon*, Hercules, Inc.) | | | | 60 | | | | | |
| Cross-linked Carboxymethylcellulose, (Aquasorb*, Hercules, Inc.) | | | | | 65 | | | | |
| Polyacrylate (Permasorb*, National Starch) | | | | | | 75 | | | |
| Starch Polyacrylate (Sanwet*, Sanyo) | | | | | | | 40 | | |
| Polyacrylate (53388, Goodrich Tire and Rubber Co.) | | | | | | | | 80 | |
| Polyacrylate (Aqua-Keep*, Mitsubishi) | | | | | | | | | 100 |

*Trademark
+ Hydrolyzed polyacrylonitrile grafted to starch

TABLE A (continued)

| Component | Amount of Components (parts by weight) Examples I | II | III | IV | V | VI | VII | VIII | IX |
|---|---|---|---|---|---|---|---|---|---|
| **Liquid Vehicle** | | | | | | | | | |
| Mineral oil, 74 cps. (Johnson & Johnson Baby Oil) | 100 | | | | | | | | |
| Mineral oil, 190 cps. | | 100 | | | | | | | |
| Triethylene glycol ester of hydrogenated rosin, 10,000 cps., softening point 10°C. (Stabelite* Ester #3, Hercules, Inc.) | | | 100 | | | | | | |
| Polymerized mixed olefins, 20,000-25,000 cps. (Wingtack* 10, Goodyear Tire and Rubber) | | | | 100 | | | | | |
| Coumarone-indene, 15,000 cps. (Cumar* NP 10, Neville Chemical) | | | | | 100 | | | | |
| Polybutene, 93 cps. | | | | | | 100 | | | |
| Polybutadiene, 30,000 cps. | | | | | | | 100 | | |
| Depolymerized natural rubber, 45,000-50,000 cps. (Isolene D, Hartman) | | | | | | | | 100 | |
| Polybutene, 494 cps. | | | | | | | | | 100 |
| **Viscosity modifier** | | | | | | | | | |
| Sodium silicate (HI-SIL*) | | 5 | | | | | | | |
| Sodium silicate (CAB-O-SIL*) | | | 5 | | | | | | |

*Trademark

EXAMPLE X

A liquid-dispersed absorbent product suitable for use without substrate is prepared by mixing 125 parts of polyacrylate (Permasorb) and 100 parts of mineral oil (74 cps.). The resulting oily product when administered on a spreading aqueous liquid quickly disperses over the surface immobilizing the aqueous liquid and simultaneously forming a gel. The substantially instantaneous spreading and formation of gel prevents undesirable water-borne damage.

EXAMPLE XI

A liquid-dispersed absorbent product is prepared by mixing 100 parts of a commercially available blend of xanthan gum, locust bean gum and guar gum (GFS, Kelco, Div. of Merck & Co.) with 100 parts of mineral oil (Johnson & Johnson Baby Oil). The product is coated on cellulose batting and the batting thus modified is used to prepare a diaper in the usual manner.

Example XII

400 parts by weight of Mitsubishi Aqua-Keep 10SH* (which is a polyacrylic acid partially neutralized with a sodium salt) is blended with 100 parts by weight of Union Carbide Sentry 600* P.E.G. (polyethylene glycol with a molecular weight of 600). The blending is carried out for 5-15 minutes in a Hobart mixer, at a medium speed, the polyethylene glycol 600 being added at a steady rate to the Aqua-Keep 10SH for approximately 2 minutes while mixing.

EXAMPLES XIII - XVI

Blended absorbent products having the compositions set forth in Table B are prepared in a similar manner to that described in Example XII.

Table B

| Ingredients | Examples | | | |
|---|---|---|---|---|
| | XIII | XIV | XV | XVI |
| Carbowax 400* Union Carbide | 100 | | | |
| Carbowax 600* Union Carbide | | 100 | | 100 |
| Carbowax 550* Union Carbide | | | 100 | |
| Sanwet 1000* (Starch polyacrylate; Sanyo) | | | | 250 |
| Aquakeep 10SH* (Polyacrylate, Mitsubishi) | 100 | 500 | 333 | |

The ingredients utilized in Examples XII - XVI have the following properties.

| | | Mol. wt. | Freezing Temp. C | Water Solubility 20°C % by wt |
|---|---|---|---|---|
| Carbowax 400 | Polyethylene Glycol | 380-420 | 4-8 | Complete |
| Carbowax 600 | Polyethylene Glycol | 570-630 | 20-25 | Complete |
| Carbowax 550 | Methoxy Polyethylene Glycol | 525-575 | 15-25 | Complete |

*Trademark

The blends produced in accordance with Examples XII - XVI are suitable for air conveying or air blasting into the interstices of a fibrous web, so as to produce an

absorbent system with rapid liquid uptake and immediate superabsorbent gelling of an added liquid such as a saline solution.

The products having the compositions identified in Table A and in Example XIII, are applied to substrates to produce absorbent articles as follows:

The products having the compositions of Examples I, IV, and VI and XII are applied respectively to (a) a nonwoven fabric sheet comprising 1.5 to 3.0 denier rayon and containing 20 to 35 percent acrylate ester copolymer binder, and having a weight of about 15 to 19 grams per square yard, (b) a nonwoven fabric sheet comprising 35 to 64 percent 1.5 to 3.0 denier polyester fiber, 14 to 40 percent 1.5 to 3.0 denier rayon fibers and 20 to 30 percent of the same acrylate ester copolymer binder, and having a weight of about 25 to 35 grams per square yard, (c) cellulosic batting to form modified absorbent materials. The materials are employed in the absorbent portion of diapers and backed with moisture impermeable film to produce disposable diapers having superior absorptive properties and in which the particulate absorbents are retained substantially in place.

The products having the compositions of Examples III and VII are employed to saturate creped paper. The modified papers are employed to produce inserts for absorbent bed paddings.

The products having the compositions of Examples V and VIII are employed to coat polyethylene film to be employed as backing for disposable diapers. The modified backing is then overlaid with fluffed cellulose fibers forming a laminate then overlaid with nonwoven fabric web to produce a superior absorbent diaper.

The products having the compositions of Examples II, IX and XII are applied to gauze fabric which is thereafter folded and cut into a size appropriate for use as bandages.

In use, each of the products, would exhibit superior absorption properties attributable to the particulate absorbent borne thereon. Further, the particulate materials are not dislodged on storage, shipping or use.

Claims:

1. An absorbent product comprising a particulate water insoluble, water swellable absorbent having a gel capacity of at least 10 dispersed in an inert organic vehicle as carrier, said vehicle having a viscosity in the range of from about 25 to about 100,000 centipoises.

2. An absorbent product comprising a particulate water insoluble, water swellable absorbent having a gel capacity of at least 10 dispersed in an inert organic vehicle as carrier, said vehicle having a viscosity in the range of from about 25 to 100,000 centipoises, there being from 2 parts to 1000 parts by weight of absorbent to every 100 parts by weight of said vehicle.

3. An absorbent product comprising a particulate water insoluble, water swellable absorbent blended with polyethylene glycol, there being from 200 parts to 1000 parts by weight of particulate absorbent to every 100 parts by weight of polyethylene glycol.

4. A product according to Claim 3 which comprises about 400 parts by weight of particulate absorbent to every 100 parts by weight of polyethylene glycol.

5. A product according to any one of Claims 1 to 4 in which the absorbent is a synthetic acrylate or an acrylate modified polysaccharide.

6. A process for preparing an absorbent article which comprises applying to the absorbing surface of such article as substrate, an absorbent product comprising particulate water insoluble, water swellable absorbent dispersed in an organic liquid vehicle as carrier, said liquid vehicle having a viscosity in the range of from about 25 to about 100,000 centipoises.

7. A bandage having enhanced absorptive properties comprising an absorbent pad bearing particulate absorbent obtained by applying to the surface thereof an absorbent product comprising a particulate water insoluble, water swellable absorbent dispersed in an inert organic vehicle as carrier, said vehicle having a viscosity in the range of from about 25 to about 100,000 centipoises.

8. An absorbent article comprising a substrate having applied thereto an absorbent product comprising a particulate water insoluble, water swellable absorbent dispersed in an inert organic vehicle, said vehicle having a viscosity in the range of from about 25 to about 100,000 centipoises.

9. An article according to Claim 8 in which the substrate is a nonabsorbent moisture impermeable diaper backing.

10. An article according to Claim 8 or Claim 9 in which the vehicle is polyethylene glycol, there being from 200 parts to 1000 parts by weight of particulate absorbent to every 100 parts by weight of said vehicle.

0157960

European Patent Office

# EUROPEAN SEARCH REPORT

Application number

EP 84 30 2059

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 001 706 (UNILEVER) * Page 4, lines 6-10; page 12, lines 1-25; page 20; claims 1,4 * | 1-10 | A 61 L 15/00 |
| | --- | | |
| X | EP-A-0 049 944 (JOHNSON & JOHNSON) * Page 5, lines 10-15; page 6, lines 14-19; page 7, lines 1-6, 21-23; page 20, table A; page 23, table B; claims 1,7,9,19 * | 1,2,5, 6-9 | |
| | --- | | |
| X | EP-A-0 009 977 (UNILEVER) * Page 4, lines 3-27; page 11, table I; claims 1,4,5,6 * | 1,2,5, 6-9 | |
| | --- | | TECHNICAL FIELDS SEARCHED (Int. Cl.4) |
| A | FR-A-1 548 038 (PROCTER & GAMBLE) * Page 9; abstract * | 1 | A 61 L |
| | --- | | |
| A | US-A-3 371 666 (A.W. LEWING) * Examples IV-VI; column 6, lines 1-34; claims 21-23 * | 1 | |
| | ----- | | |

The present search report has been drawn up for all claims

| Place of search | Date of completion of the search | Examiner |
|---|---|---|
| THE HAGUE | 27-11-1984 | PELTRE CHR. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82